# EUROPÄISCHE PATENTSCHRIFT

(11) **EP 2 689 716 B1**
(45) Veröffentlichungstag und Bekanntmachung des Hinweises auf die Patenterteilung: **25.04.2018**
(21) Anmeldenummer: 13178060.3
(22) Anmeldetag: 25.07.2013
(51) Int. Cl.: A61B 1/005, A61B 1/273

(54) **Endoskop**
Endoscope
Endoscope

(30) Priorität: 25.07.2012 DE 102012106755
(43) Veröffentlichungstag der Anmeldung: 29.01.2014
(73) Patentinhaber: Karl Storz SE & Co. KG, 78532 Tuttlingen (DE)
(72) Erfinder: Schwarz, Peter, 78532 Tuttlingen-Nendingen (DE)
(74) Vertreter: Witte, Weller & Partner Patentanwälte mbB

(56) Entgegenhaltungen:
- EP-A1- 1 825 801
- EP-A1- 2 324 755
- WO-A1-2012/063880
- US-A- 5 060 632
- US-A1- 2010 210 908

## Beschreibung

Die vorliegende Erfindung betrifft ein endoskopisches Instrument mit einem Bedienstück mit einem Bedienelement, einem Instrumentenschaft mit einem betätigbaren Abschnitt, wobei der Instrumentenschaft mit dem Bedienstück verbunden ist, einem Stellelement mit einer ersten Rotationsachse, einem Zugelement, das mit dem Stellelement und dem Abschnitt des Instrumentenschafts mechanisch gekoppelt ist, so dass eine Verlagerung des Stellelements eine Betätigung des Abschnitts mittels einer Kraftübertragung über das Zugelement bewirken kann, und einem Aktuator, der mit dem Stellelement gekoppelt ist, so dass eine Betätigung des Aktuators eine Verlagerung des Stellelements durch Kraftübertragung vom Aktuator auf das Stellelement bewirken kann, wobei der Aktuator eine zweite Rotationsachse aufweist, wobei das Bedienelement dafür ausgebildet ist, dass ein Benutzer durch Betätigen des Bedienelements eine Verlagerung des Stellelements bewirken kann, wobei das Bedienelement mit dem Stellelement derart mechanisch gekoppelt ist, dass eine durch den Benutzer auf das Bedienelement ausgeübte Kraft zumindest teilweise auf das Stellelement übertragen und die Verlagerung des Stellelements bewirkt werden kann.

Ein solches Endoskop ist aus US 4,499,895 bekannt.

Endoskopische Instrumente mit flexiblem oder starrem Endoskopschaft werden sowohl in der Industrie als auch im medizinischen Bereich verwendet. Beispielsweise werden flexible Endoskope im veterinär-medizinischen Bereich bei der gastroendoskopischen Untersuchung von Großtieren eingesetzt. Diese Endoskope besitzen häufig an einem distalen Ende ihres Endoskopschafts einen distalen Endabschnitt, der in einem Endstück endet. Das Endstück stellt den distalen Teil des Endoskops dar, der in den zu untersuchenden Körper eingeführt wird. Es weist üblicherweise das distale Ende einer Endoskopoptik sowie zum Teil auch Absaug-, Spül- und Werkzeugkanäle auf.

Um eine größtmögliche Flexibilität bezüglich einer räumlichen Ausrichtung des Endstücks bei der Untersuchung gewährleisten zu können, ist der Endabschnitt des Endoskopschafts üblicherweise ablenkbar ausgebildet. Durch ein Ablenken, bzw. allgemein Betätigen, des Abschnitts zu dem restlichen Instrumentenschaft, insbesondere durch ein Abwinkeln oder Abkrümmen, kann ein Teil des Instrumentenschafts, insbesondere das Endstück, nach Wunsch ausgerichtet werden. Bei diesem Ablenken ist eine vorsichtige Vorgehensweise eines Anwenders notwendig, damit kein Gewebe verletzt wird, das sich um den Endabschnitt herum befindet. Es ist daher wichtig, dass das Betätigen des Abschnitts sehr präzise steuerbar ist.

Die Ablenkung des Abschnitts erfolgt bei flexiblen und starren Endoskopen gemäß dem Stand der Technik über ein Zugelement, insbesondere über Bowdenzüge. Das Zugelement ist mit einem Stellelement, insbesondere einem Lenkgetriebe, verbunden. Das Zugelement ist dabei häufig an einem Schnurrad befestigt. Durch eine Betätigung des Bedienelements am Bedienstück des Endoskops wird das Stellelement bewegt. Durch eine rotatorische Bewegung des Stellelements ergibt sich eine translatorische Bewegung des Zugelements. Die Bewegung des Zugelements hat wiederum eine Ablenkung des Abschnitts zur Folge.

Die eingangs genannte US 4,499,895 zeigt ein elektrisches Endoskop, dessen Bedienung über einen Hebel erfolgt, der mit dem Stellelement gekoppelt ist. Wenn der Hebel gegenüber dem Stellelement verlagert wird, wird eine Biegung des Hebels oder eine Widerstandsänderung eines Potentiometers erfasst und dadurch eine Unterstützung des Stellelements durch einen Aktuator eingestellt. Der gezeigte Aufbau ist allerdings sehr unhandlich und erfordert im Fehlerfall einen speziellen Lösemechanismus, um das Endoskop noch bedienen zu können.

US 7,331,924 beschreibt ein sogenanntes elektrisches Endoskop mit einem ablenkbaren distalen Abschnitt. Die Bedienung des Endoskops erfolgt hier über einen Trackball, dessen Verlagerung durch den Daumen oder Finger eines Benutzers von einer elektrischen Schaltung erfasst wird. In Abhängigkeit von der rotatorischen Verlagerung des Trackballs, wie sie vom Benutzer gewählt wird, wird in einer Ablenkungssteuerung die Ansteuerung eines Motors bestimmt, der eine Verlagerung des Zugelements und damit ein Ablenken des distalen Abschnitts bewirkt.

Der Nachteil eines solchen elektrischen Endoskops ist jedoch, dass die Bedienung als weniger intuitiv empfunden wird, da die Bedienung eines elektrischen Endoskops, z.B. beim Anstoß an Gewebe, nicht die Rückmeldung liefert, die der Benutzer von mechanischen Endoskopen kennt. Dieselbe Problematik ergibt sich auch bei elektrischen Endoskopen, die über einen Joystick bedient werden. Dabei sei lediglich beispielsweise auf das Dokument US 6,932,761 hingewiesen. Elektrische Endoskope haben außerdem den Nachteil, dass sie sich bei einem Defekt möglicherweise nur schwer aus dem Hohlraum herausziehen lassen, wenn der distale Abschnitt abgelenkt ist.

Bei mechanischen Endoskopen erfolgt die Ablenkung des Abschnitts ausschließlich über eine mechanische Krafteinwirkung des Benutzers auf eine außen liegende Handhabe am Bedienstück des Endoskops. Dafür ist das Stellelement üblicherweise fest auf einer Welle der Handhabe angeordnet. Betätigt der Benutzer die Handhabe, so entsteht eine rotatorische Verlagerung des Stellelements und dadurch wiederum die translatorische Bewegung des Zugelements.

Rein mechanische Endoskope können aber je nach Länge des Endoskops und der Lage des Endoskopschafts erhebliche Kräfte für eine Betätigung erfordern. Zudem erzeugt die mechanische Betätigung des Abschnitts automatisch eine gewisse Rückstellkraft in Richtung der nicht-abgelenkten Position (Nullposition) des Endoskops. Ferner bildet der Abschnitt bei der Ablenkung mit dem Zugelement ein Feder-Dämpfer-System, das Energie beim Spannen speichert und beim Entspannen freigibt. Dadurch können eine Anlauftotzeit oder ein Nachlaufweg beim Ablenken des Endabschnitts entstehen. Dies führt dazu, dass der Benutzer die Ablenkung des Abschnitts, die er eigentlich einstellen möchte, um eine bestimmte Stelle betrachten zu können, nur näherungsweise oder iterativ einstellen kann.
US 2010/0210908 A1 offenbart eine Bedieneinrichtung, bei der ein Handgriff mittels einer flexiblen Verbindungsröhre mit einem Stellelement verbunden ist. Das Stellelement ist mit einem Elektromotors gekoppelt. Wird der Handgriff gedreht, so entsteht ein Drehmoment in der Verbindungsröhre, das erfasst wird und bei einer Ansteuerung des Elektromotors berücksichtigt werden kann.

WO 2012/063880 A1 offenbart eine Bedieneinrichtung, die wahlweise mit einer manuellen Handhabe oder mit einem Motorantrieb betätigt werden kann. Hierfür ist eine gemeinsame Schnittstelle vorgesehen, auf die sowohl die Handhabe als auch der Motorantrieb gesetzt werden kann. Bei einer anderen Ausgestaltung ist eine Handhabe mit einem Stellelement und über ein Getriebe mit einem Elektromotor gekoppelt. Innerhalb des Getriebes wird ein Drehmoment ermittelt, das bei einer Ansteuerung des Elektromotors berücksichtigt werden kann.

EP 1 825 801 A1 offenbart eine Bedieneinrichtung, die wahlweise mit einer manuellen Handhabe oder mit einem Motorantrieb betätigt werden kann. Hierfür ist eine gemeinsame Schnittstelle für eine formschlüssige Verbindung vorgesehen, auf die sowohl die Handhabe als auch der Motorantrieb gesetzt werden kann.

Es ist daher eine Aufgabe der vorliegenden Erfindung, ein endoskopisches Instrument der eingangs genannten Art dahingehend weiterzubilden, dass der Benutzer zwar eine Unterstützung, wie eine elektrische Betätigung, bei der Bedienung erhält, dabei aber nicht die mechanische Rückmeldung des Endoskops verliert. Außerdem ist es eine Aufgabe, dass das Endoskop selbst bei einem Ausfall der elektrischen Steuerung weiterhin bedienbar bleibt.

Erfindungsgemäß wird die Aufgabe durch die Merkmale des kennzeichnenden Teils des Anspruchs 1 bzw. des Anspruchs 2 gelöst.

Eine Besonderheit der Erfindung liegt darin, dass aufgrund der Anordnung der ersten und zweiten Rotationsachse eine motorisch unterstützte Bedienung des Endoskops möglich ist, im Fehlerfall aber auch ohne einen zusätzlichen Lösemechanismus eine rein manuelle Bedienung möglich ist.

Dabei ist es insbesondere vorteilhaft, wenn der Winkel zwischen erster und zweiter Rotationsachse kleiner als 85° ist, bevorzugt kleiner 75° ist, besonders bevorzugt kleiner als 60° und insbesondere kleiner gleich 45° ist. Werden für eine Wirkverbindung zwischen Aktuator und Stellelement Zahnräder verwendet, so lässt sich der Winkel insbesondere über eine Schrägverzahnung einstellen. Bei einigen Ausgestaltungen ist es besonders vorteilhaft, wenn der Winkel zwischen erster und zweiter Rotationsachse zumindest in etwa 0° beträgt, die Achsen also zumindest in etwa parallel zueinander verlaufen, oder die Achsen sogar zusammenfallen.

Eine weitere Besonderheit der Erfindung liegt darin, dass das Stellelement sowohl mit dem Aktuator, insbesondere einem Elektromotor, und dem Bedienelement so gekoppelt ist, dass sowohl eine vom Aktuator als auch vom Bedienelement bereitgestellte Kraft auf das Stellelement übertragen und die Verlagerung des Stellelements bewirkt werden kann.

Im Normalbetrieb betätigt der Benutzer das Bedienelement, um eine Betätigung des Abschnitts zu bewirken. Die Verlagerung des Bedienelements wird detektiert, und der Aktuator wird so angesteuert, dass er die vom Bedienelement hervorgerufene Verlagerung des Stellelements unterstützt. Findet keine weitere Verlagerung des Bedienelements statt, so findet auch keine weitere Verlagerung des Stellelements durch den Aktuator statt.

Fällt der Aktuator aufgrund eines Defekts aus, so bleibt das Endoskop dennoch weiterhin bedienbar, da über das Bedienelement weiterhin eine Kraft auf das Stellelement ausgeübt werden kann. Somit bleibt auch im Fehlerfall die Möglichkeit einer Betätigung des Abschnitts weiterhin erhalten. Es fehlt dann lediglich die Unterstützung durch den Aktuator.

Der Aktuator ist bevorzugt im Bedienstück des Endoskops angeordnet. Das Stellelement ist bevorzugt im Bedienstück des Endoskops angeordnet. Das Zugelement ist bevorzugt als ein Bowdenzug ausgeführt, der insbesondere um das Stellelement herum geführt ist. Das Stellelement ist bevorzugt als Schnurrad ausgeführt. Bei dem Aktuator handelt es sich bevorzugt um einen Elektromotor, der insbesondere ein Zahnrad oder ein Getriebe aufweist.

Wenn im Rahmen der Erläuterungen von einer Betätigung oder einer Verlagerung gesprochen wird, so soll darunter bevorzugt eine drehende Betätigung und/oder eine rotatorische Verlagerung verstanden werden. Es sind aber bei Verwendung von entsprechenden Reglern oder Schiebern auch Ausführungsformen möglich, bei denen eine translatorische oder lineare Betätigung oder Verlagerung stattfindet. Die jeweilige Kraftübertragung zwischen dem Bedienelement und dem Stellelement bzw. dem Aktuator und dem Stellelement kann über einen oder mehrere Punkte erfolgen, an denen jeweils eine Kraft oder ein resultierendes Drehmoment übertragen werden.

Bevorzugt handelt es sich bei dem endoskopischen Instrument um ein Endoskop, wobei der Instrumentenschaft dann als Endoskopschaft ausgebildet ist. Ferner ist es bevorzugt, wenn der betätigbare Abschnitt ein ablenkbarer distaler Abschnitt ist. Der Begriff der Betätigung bzw. des Betätigens umfasst insbesondere ein Schneiden, Klemmen und Drehen, sowie besonders bevorzugt ein Ablenken. Ferner kann es bevorzugt sein, das Stellelement und das Zugelement einstückig auszuführen, insbesondere bei einer Zange oder Ähnlichem, wo Stellelement und Zugelement durch eine Zug-Druckstange gebildet sind.

Bei einer bevorzugten Ausgestaltung weist das endoskopische Instrument ein zweites Stellelement, ein zweites Zugelement, das mit dem zweiten Stellelement und dem Abschnitt des Instrumentenschafts mechanisch gekoppelt ist, so dass eine Verlagerung des zweiten Stellelements eine Betätigung des Abschnitts mittels eines Kraftübertragung über das zweite Zugelement bewirken kann, und einen zweiten Aktuator auf, der mit dem zweiten Stellelement gekoppelt ist, so dass eine Betätigung des zweiten Aktuators eine Verlagerung des zweiten Stellelements durch Kraftübertragung vom zweiten Aktuator auf das zweite Stellelement bewirken kann, wobei das Bedienelement auch dafür ausgebildet ist, dass ein Benutzer durch Betätigen des Bedienelements eine Verlagerung des zweiten Stellelements bewirken kann, und wobei das Bedienelement mit dem zweiten Stellelement derart mechanisch gekoppelt ist, dass eine durch den Benutzer auf das Bedienelement ausgeübte Kraft zumindest teilweise auf das zweite Stellelement übertragen werden und die Verlagerung des zweiten Stellelements bewirken kann.

Bei dieser Ausgestaltung weist das Bedienelement bevorzugt eine erste Handhabe, die das genannte, erste Stellelement verlagert, und eine zweite Handhabe auf, die das zweite Stellelement verlagert. Dabei führen eine Betätigung der ersten Handhabe zu einer ersten Bewegung des Abschnitts, insbesondere zu einer Bewegung links/rechts, und eine Betätigung der zweiten Handhabe zu einer zweiten Bewegung des Abschnitts, insbesondere zu einer Bewegung auf/ab. Bevorzugt ist die eine Handhabe auf einer Hohlwelle angeordnet, in der eine Welle der anderen Handhabe angeordnet ist.

Damit ist die Aufgabe vollständig gelöst.

Bei einer bevorzugten Ausführungsform weist das Stellelement einen ersten Zahnkranz auf, weist der Aktuator einen zweiten Zahnkranz auf, und sind der erste und der zweite Zahnkranz kraftschlüssig gekoppelt, insbesondere derart angeordnet, dass sie im Eingriff miteinander stehen.

Diese Ausgestaltung ermöglicht gerade im Hinblick auf eine rotatorische Verlagerung des Stellelements eine besonders gute Kopplung zwischen Stellelement und Aktuator. Außerdem ist eine solche Kopplung mechanisch zuverlässig und dauerhaft stabil.

Bei einer anderen bevorzugten Ausführungsform sind das Stellelement und der Aktuator über einen Riemen, einen Zahnriemen, über eine Kette oder als Reibräder miteinander verbunden.

Bei einer weiteren bevorzugten Ausgestaltung der Erfindung weist das Stellelement einen ersten Zahnkranz auf, weist das Bedienelement einen dritten Zahnkranz auf, und sind erster und dritter Zahnkranz kraftschlüssig gekoppelt, insbesondere derart angeordnet, dass sie in Eingriff miteinander stehen.

Diese Ausgestaltung ermöglicht gerade im Hinblick auf eine rotatorische Verlagerung des Stellelements eine besonders gute Kopplung zwischen Stellelement und Bedienelement. Außerdem ist eine solche Kopplung mechanisch zuverlässig und dauerhaft stabil.

Bei einer weiteren bevorzugten Ausführungsform sind das Bedienelement und der Aktuator derart miteinander mechanisch wirkverbunden, dass die durch den Benutzer auf das Bedienelement ausgeübte Kraft zumindest teilweise auf den Aktuator übertragen werden kann.

Bei dieser Ausgestaltung besteht eine mechanische Kopplung von Bedienelement, Stellelement und Aktuator untereinander. Auf diese Weise ist sichergestellt, dass der Aktuator und das Bedienelement gleichzeitig auf das Stellelement eine Kraft übertragen können. Im Falle eines Defekts des Aktuators führt zwar eine Betätigung des Bedienelements - ohne weitere konstruktive Maßnahmen - auch zu einer Verlagerung des Aktuators, was zu einem gewissen Widerstand bei der Verlagerung des Stellelements führt. Durch eine geeignete Auswahl des Aktuators und/oder durch weitere technische Maßnahmen, insbesondere eine Kupplung zwischen Aktuator und Stellelement, kann dieser Einfluss aber gering bzw. vernachlässigbar gehalten werden.

Bei einer weiteren bevorzugten Ausführungsform ist eine Steuerung dafür ausgebildet, den Aktuator so ansteuern zu können, dass er gegen den Anwender, insbesondere gegen die Handhabe, eine Kraft ausübt. Dies ermöglicht eine Kommunikation mit dem Anwender. Beispielsweise können dem Anwender bestimmte Positionen oder Zustände durch unterschiedliches Vibrieren des Aktuators angezeigt werden. Der Aktuator zeigt dem Anwender bevorzugt durch einen kurzen Bremsimpuls die Neutralstellung des Bedienhebels an. In diesem Zusammenhang ist es vorteilhaft, dass dem Stellelement ein Winkelmesser zugeordnet ist, um die Neutralstellung zu registrieren. Ferner ist es bevorzugt, wenn der Aktuator so angesteuert wird, dass er durch ein Vibrieren die Überschreitung einer Ablenkkraftschwelle anzeigt. Außerdem ist es bevorzugt, wenn externe Systeme Informationen über einen sicherheitskritischen Zustand übermitteln (zum Beispiel zu hoher Insufflationsdruck oder Überschreiten einer Temperatur) und dies mittels einer entsprechenden Ansteuerung dem Anwender über unterschiedliche Vibrationsfrequenzen mitgeteilt wird.

Bei einer weiteren vorteilhaften Ausgestaltung der Erfindung weist das Bedienelement eine Handhabe und ein Mitnehmerelement auf, sind die Handhabe und das Mitnehmerelement so angeordnet, dass sie relativ zueinander verlagert werden können, und sind Handhabe und Mitnehmerelement so miteinander gekoppelt, dass eine Verlagerung der Handhabe eine Verlagerung des Mitnehmerelements bewirken kann.

Bei dieser Ausgestaltung steht bevorzugt das Mitnehmerelement in Wirkverbindung zum Stellelement. Da die Handhabe und das Mitnehmerelement relativ zueinander verlagert werden können, kann auf einfache Weise festgestellt werden, ob der Benutzer gerade eine Kraft auf die Handhabe ausübt, um die Betätigung des Abschnitts zu steuern. Wird eine solche relative Verlagerung erkannt, wird bevorzugt die entsprechende Ansteuerung des Aktuators eingeleitet.

Unter dem Begriff "entsprechende Ansteuerung" soll verstanden werden, dass der Aktuator die vom Benutzer gewünschte Aktion unterstützt. Wenn der Benutzer das Stellelement in eine bestimmte Richtung, z.B. im Uhrzeigersinn, verlagern möchte, so unterstützt der Aktuator diese Verlagerung in derselben Richtung. Wenn der Benutzer das Stellelement in eine andere Richtung, z.B. gegen den Uhrzeigersinn, verlagern möchte, so unterstützt der Aktuator diese Verlagerung in dieser selben Richtung.

Die relative Verlagerung von Handhabe und Mitnehmerelement zueinander bedeutet dabei insbesondere, dass die Handhabe eine zumindest geringfügige rotatorische Verlagerung erfährt, die das Mitnehmerelement nicht zeitgleich und/oder nicht in gleicher Weise erfährt. Dabei ist es insbesondere bevorzugt, dass die Handhabe und das Mitnehmerelement entlang derselben Achse und/oder Welle angeordnet sind. Ferner ist es bevorzugt, dass die Handhabe und das Mitnehmerelement fest auf derselben Welle angeordnet sind, und sich die Möglichkeit der relativen Verlagerung durch ein zumindest geringfügiges Verdrehen der Welle in einem Bereich zwischen der Position der Handhabe und der Position des Mitnehmerelements ergibt.

Es ist aber besonders bevorzugt, dass die Handhabe und das Mitnehmerelement nicht über eine Welle starr miteinander verbunden sind, sondern die Handhabe lediglich konzentrisch ohne eine starre Verbindung zur Welle des Mitnehmerelements angeordnet ist oder konzentrisch zu einer gedachten Verlängerung dieser Welle angeordnet ist. In diesem Fall wird insbesondere durch eine lose mechanische Kopplung von Handhabe und Mitnehmerelement sichergestellt, dass, zumindest nach einer bestimmten relativen Verlagerung von Handhabe und Mitnehmerelement zueinander, die Verlagerung der Handhabe eine Verlagerung des Mitnehmerelements bewirkt.

Die relative Verlagerung ist dabei vorzugsweise auf maximal 10°, bevorzugt maximal 3°, besonders bevorzugt maximal 1° und insbesondere bevorzugt maximal 0,3° begrenzt. Dies erfolgt bevorzugt durch die nachfolgend beschriebene Koppeleinrichtung.

Die Handhabe ist bevorzugt als Rad, Hebel oder Kurbel ausgeführt. Außerdem ist es bevorzugt, dass die Handhabe und das Mitnehmerelement als separate Bauteile ausgeführt sind, nicht zusammen als einstückiges Bauteil. Die relative Verlagerung ergibt sich bervorzugt durch eine rotatorische Verlagerung der Bauteile zueinander, insbesondere ohne Handhabe oder Mitnehmerelement zu biegen oder zu verdrehen.

Bei einer weiteren vorteilhaften Ausgestaltung der Erfindung weist das Bedienelement eine Koppeleinrichtung auf, die einen ersten Teil und einen zweiten Teil aufweist, wobei einer der Teile mit einer Handhabe verbunden ist und der andere Teil mit dem Stellelement oder dem Mitnehmerelement verbunden ist, wobei die Teile so ausgebildet und angeordnet sind, dass eine Kraftübertragung zwischen Handhabe und Stellelement oder Handhabe und Mitnehmerelement aufgrund einer Kraftübertragung zwischen dem ersten und dem zweiten Teil erfolgen kann.

Diese Ausgestaltung ermöglicht es, dass die Handhabe gegenüber dem Stellelement oder gegenüber dem Mitnehmerelement zumindest geringfügig verdreht werden kann und dabei eine Kraftübertragung von der Handhabe auf das Stellelement oder das Mitnehmerelement erfolgen kann. Insbesondere kann eine Kraft, die eine rotatorische Verlagerung der Handhabe bewirkt, zumindest teilweise auf das Stellelement oder Mitnehmerelement übertragen werden, so dass eine rotatorische Verlagerung des Stellelements oder des Mitnehmerelements erfolgt.

Die Kopplung erfolgt bei einer bevorzugten Ausführungsform durch eine Kombination einer Nut und eines Vorsprungs, der in der Nut läuft, wobei die Nut an der Handhabe oder an dem Stellelement oder Mitnehmerelement angeordnet ist und der Vorsprung an dem jeweiligen anderen Element angeordnet ist. Solange sich der Vorsprung zwischen den beiden Enden der Nut bewegt, können sich die Handhabe und das Stellelement oder Mitnehmerelement relativ zueinander verlagern. Drückt der Vorsprung gegen eines der Enden der Nut, so bewirkt die Verlagerung der Handhabe eine Verlagerung des Stellelements oder Mitnehmerelements. Es ist bevorzugt, dass die Handhabe und das Stellelement oder Mitnehmerelement jeweils eine rotatorische Verlagerung ausführen, wobei die Nut dann die Form eines Kreisbogens hat.

Bei einer weiteren vorteilhaften Ausgestaltung der Erfindung weist die Koppeleinrichtung eine Kraftmesseinrichtung auf, die dafür ausgebildet ist, anzuzeigen, ob eine Kraft zwischen dem ersten Teil und dem zweiten Teil ausgeübt wird, oder eine Indikation über die Größe einer zwischen dem ersten Teil und dem zweiten Teil ausgeübten Kraft zu geben, insbesondere auch eine Indikation über die Richtung der ausgeübten Kraft zu geben.

Bei dieser Ausgestaltung kann auf einfache Weise ermittelt werden, ob der Benutzer die Handhabe verlagert, um eine Kraft über das Mitnehmerelement auf das Stellelement zu übertragen. Wird eine solche Kraft registriert, wird vorzugsweise der Aktuator betätigt, um die gewünschte Verlagerung des Stellelements zu unterstützen. Dabei ist es bevorzugt, dass die Kraft vorzeichenbehaftet erfasst wird, beispielsweise vorwärts/rückwärts oder im/gegen den Uhrzeigersinn, damit der Aktuator eine Verlagerung des Stellelements in zwei Richtungen unterstützen kann.

Dabei ist es insbesondere bevorzugt, dass die Kraftmesseinrichtung eine Indikation über die Größe der zwischen dem ersten Teil und dem zweiten Teil ausgeübten Kraft gibt. Zeigt die Indikation an, dass die Kraft gering ist, so liefert der Aktuator auch nur eine geringe Unterstützung. Zeigt die Indikation hingegen an, dass die Kraft groß ist, so liefert der Aktuator eine große Kraft zur Unterstützung.

Die Begriffe "große Unterstützung" und "geringe Unterstützung" sollen vorzugsweise im Hinblick auf die Kraft verstanden werden, die der Aktuator auf das Stellelement ausübt. Wenn es sich bei dem Aktuator bevorzugt um einen Elektromotor handelt, so wird der Elektromotor bei einer großen Unterstützung mit einem größeren Strom gespeist als bei einer geringen Unterstützung.

Die Indikation ist bevorzugt einfach gehalten, insbesondere eine Unterscheidung zwischen "keine Kraft", "geringe Kraft" und "große Kraft", da dies eine besonders preisgünstige und robuste Fertigung der Kraftmesseinrichtung erlaubt. Bei anderen Ausgestaltungen ist es bevorzugt, dass die Indikation in mehr als drei, bevorzugt mehr als fünf, besonders bevorzugt mehr als zehn Stufen und insbesondere stufenlos gegeben wird. In Abhängigkeit von der Größe der Kraft erfolgt eine entsprechende Unterstützung durch den Aktuator.

Die Kraftmessung erfolgt bevorzugt unter Verwendung eines Drucksensors.

Bei einer weiteren vorteilhaften Ausgestaltung der Erfindung ist am ersten und/oder am zweiten Teil eine Federeinrichtung angeordnet, so dass eine Kraft zwischen dem ersten Teil und dem zweiten Teil über die Federeinrichtung übertragen wird.

Diese Ausgestaltung ermöglicht eine besonders intuitive Bedienführung, da der Benutzer die Handhabe gegen einen Widerstand der Federeinrichtung verlagert und so eine gefühlte Rückmeldung bei der Betätigung der Handhabe erfährt. Insbesondere kann der Benutzer spüren, wie stark der Aktuator aufgrund der Verlagerung der Handhabe betätigt werden wird. Der Benutzer kann so die Betätigung des Abschnitts gezielt einstellen.

Bei einer weiteren vorteilhaften Ausgestaltung der Erfindung weist der erste Teil zwei Elemente auf und ist der zweite Teil als Vorsprung ausgebildet, der zwischen den zwei Elementen angeordnet ist, und sind der erste und der zweite Teil zueinander so angeordnet, dass eine Verlagerung des ersten Teils eine Verlagerung des zweiten Teils bewirken kann.

Bei dieser Ausgestaltung besteht ein gewisses Spiel, innerhalb dessen sich der Vorsprung zwischen den zwei Elementen, die vorzugsweise als hervorstehende Elemente ausgeführt sind, bewegen kann. Stößt der zweite Teil bei seiner Verlagerung gegen ein Element des ersten Teils, so findet eine gemeinsame Verlagerung von erstem und zweitem Teil statt. Auf diese Weise ist eine relative Verlagerung von Handhabe und Mitnehmerelement möglich. Die Verlagerung kann auch einfach detektiert werden und eine entsprechende Ansteuerung des Aktuators erfolgen.

Bei einer weiteren vorteilhaften Ausgestaltung der Erfindung weist das Endoskop einen ersten Winkelmesser auf, der dafür ausgebildet ist, eine Indikation über einen ersten Stellwinkel einer Handhabe des Bedienelements zu geben.

Bei dieser Ausgestaltung ist eine Ansteuerung des Aktuators besonders gut zu realisieren. Beispielsweise kann in einer Tabelle abgelegt sein, welche Betätigung der Aktuator erfahren muss, insbesondere welchen Drehwinkel der Aktuator durchführen muss, damit das Stellelement in die Position gelangt, die der Benutzer anhand des an der Handhabe eingestellten ersten Stellwinkels erzielen möchte. Es ist nicht erforderlich, den ersten Stellwinkel direkt an der Handhabe zu erfassen. Vielmehr ist es ausreichend, wenn ein Stellwinkel eines Elements gemessen, das einer Rotation der Handhabe folgt, insbesondere aufgrund einer mechanischen Verbindung über Achsen oder Zahnräder, so dass der Stellwinkel dieses Elements eine Indikation über den ersten Stellwinkel gibt.

Ferner kann anhand einer Erfassung von mindestens zwei ersten Stellwinkeln zu verschiedenen Zeiten eine Änderung des ersten Stellwinkels errechnet werden und die Änderung des ersten Stellwinkels zur Ansteuerung des Aktuators verwendet werden. Dabei ist es bevorzugt, außerdem das Zeitintervall zwischen den Messungen zu erfassen, um anhand der Division der Änderung des ersten Stellwinkels durch das Zeitintervall zwischen den zwei Messungen die Winkelgeschwindigkeit bestimmen zu können. Dabei ist es bevorzugt, eine große Unterstützung durch den Aktuator bereitzustellen, wenn die Winkelgeschwindigkeit groß ist, und eine geringe Unterstützung durch den Aktuator bereitzustellen, wenn die Winkelgeschwindigkeit gering ist.

Bei einer weiteren vorteilhaften Ausgestaltung der Erfindung weist das Endoskop ferner einen zweiten Winkelmesser auf, der dafür ausgebildet ist, eine Indikation über einen zweiten Stellwinkel des Stellelements zu geben.

Auch mit dieser Ausgestaltung lässt sich eine Ansteuerung des Aktuators besonders gut zu realisieren. Wenn der zweite Stellwinkel des Stellelements bekannt ist, kann daraus einerseits eine Differenz des zweiten Stellwinkels zum ersten Stellwinkel und so die erforderliche Betätigung des Aktuators ermittelt werden. Auch hier werden bevorzugt Winkeländerung und/oder Winkelgeschwindigkeit bei der Ansteuerung des Aktuators berücksichtigt. Es ist nicht erforderlich, den zweiten Stellwinkel direkt am Stellelement zu erfassen. Vielmehr ist es ausreichend, wenn ein Stellwinkel eines Elements gemessen, das einer Rotation des Stellelements folgt, insbesondere aufgrund einer mechanischen Verbindung über Achsen oder Zahnräder, so dass der Stellwinkel dieses Elements eine Indikation über den zweiten Stellwinkel gibt.

Bei einer weiteren vorteilhaften Ausgestaltung der Erfindung weist das Endoskop ferner einen dritten Winkelmesser auf, der dafür ausgebildet ist, eine Indikation über einen dritten Stellwinkel des Aktuators zu geben.

Bei einer weiteren vorteilhaften Ausgestaltung der Erfindung weist das Endoskop ferner einen vierten Winkelmesser auf, der dafür ausgebildet ist, eine Indikation über einen vierten Stellwinkel eines Mitnehmerelements zu geben.

Auch bei den letztgenannten Ausführungsformen gilt in gleicher Weise, wie oben erläutert, dass es nicht erforderlich ist, den dritten Stellwinkel direkt am Aktuator und/oder den vierten Stellwinkel direkt am Mitnehmerelement zu erfassen. Ferner sei darauf hingewiesen, dass zwei oder mehr der genannten Winkelmesser kombiniert werden können. Außerdem gelten die Erläuterungen im Zusammenhang mit dem ersten und dem zweiten Stellwinkel sinngemäß auch für den dritten und vierten Stellwinkel.

Bei einer weiteren vorteilhaften Ausgestaltung der Erfindung weist das Endoskop eine Steuereinrichtung auf, die dafür ausgebildet ist, unter Berücksichtigung eines ersten Stellwinkels einer Handhabe des Bedienelements und eines zweiten Stellwinkels eines Stellelements den Aktuator anzusteuern.

Bei dieser Ausgestaltung wird eine Abweichung zwischen dem ersten Stellwinkel der Handhabe und dem zweiten Stellwinkel des Stellelements ermittelt. Wird hier ein Unterschied festgestellt, so zeigt dies an, dass der Benutzer die Handhabe gegenüber dem Stellelement verlagert hat und eine Verlagerung des Stellelements erzielen möchte. In diesem Fall wird der Aktuator zur Unterstützung der Verlagerung des Stellelements betätigt. Dabei wird bevorzugt die Größe der Abweichung zwischen erstem Stellwinkel und zweitem Stellwinkel bei der Ansteuerung des Aktuators berücksichtigt.

Ist die Abweichung groß, so erfolgt eine große Unterstützung durch den Aktuator. Ist die Abweichung gering, so erfolgt eine geringe Unterstützung durch den Aktuator. Wird auch eine Winkelgeschwindigkeit berücksichtigt, wie bereits zuvor erläutert, ist es bevorzugt, eine große Unterstützung durch den Aktuator bereitzustellen, wenn die Winkelgeschwindigkeit groß ist, und eine geringe Unterstützung durch den Aktuator bereitzustellen, wenn die Winkelgeschwindigkeit gering ist.

Bei einer weiteren vorteilhaften Ausgestaltung der Erfindung weist das Endoskop ferner eine Steuereinrichtung auf, die dafür ausgebildet ist, den Aktuator so anzusteuern, dass das Stellelement in einer vorgegebenen Position gehalten wird, wenn der Benutzer keine Kraft auf das Bedienelement ausübt.

Wie bereits eingangs erläutert, stellt der distale Abschnitt mit dem Zugelement ein Feder-Dämpfer-System dar. Beendet ein Benutzer die Betätigung an der Handhabe, so besteht die Tendenz, dass sich das System etwas entspannt und die Betätigung des Abschnitts verändert wird. Bei der vorgeschlagenen Ausführungsform wirkt der Aktuator dieser Tendenz entgegen. Wird von dem Benutzer keine Kraft auf das Bedienelement ausgeübt, so stellt der Aktuator eine Kraft zur Verfügung, die das Stellelement in der durch den Benutzer vorgegebenen Position hält. Die Feststellung, dass der Benutzer keine Kraft auf das Bedienelement ausübt, findet bevorzugt unter Verwendung der Kraftmesseinrichtung statt. Besonders bevorzugt ist es, eine Gleichheit des ersten Stellwinkels und des zweiten Stellwinkels innerhalb eines vorgegebenen Toleranzbereichs als Anzeichen dafür zu interpretieren, dass der Benutzer keine Kraft auf das Bedienelement ausübt.

Bei einer weiteren vorteilhaften Ausgestaltung der Erfindung weist das Endoskop eine Steuereinrichtung auf, die dafür ausgebildet ist, den Aktuator so anzusteuern, dass er auf das Stellelement eine Kraft ausübt, die zumindest bereichsweise zumindest ungefähr proportional zu der Kraft ist, die der Benutzer auf das Bedienelement ausübt.

Bei dieser Ausgestaltung lässt es sich auf einfache Weise realisieren, dass die vom Benutzer auf das Bedienelement ausgeübte Kraft zu einer entsprechenden Unterstützung durch den Aktuator führt. Die Messung der Kraft erfolgt bevorzugt über eine Kraftmesseinrichtung. Besonders bevorzugt wird eine Differenz zwischen dem ersten Stellwinkel und dem zweiten Stellwinkel als eine Indikation für die vom Benutzer aufgebrachte Kraft benutzt.

Bei einer weiteren vorteilhaften Ausgestaltung der Erfindung fallen die erste und zweite Rotationsachse zusammen, insbesondere ist der Aktuator mit dem Stellelement starr auf einer gemeinsamen Achse gekoppelt.

Diese Ausgestaltung ermöglicht eine besonders einfache Ausführung.

Bei einer weiteren vorteilhaften Ausgestaltung der Erfindung sind eine Handhabe und das Stellelement auf einer gemeinsamen Achse angeordnet, die von einer Rotationsachse des Aktuators verschieden ist.

Diese Ausgestaltung ermöglicht es auf einfache Weise, der Rotationsbewegung des Aktuators und der Rotationsbewegung des Stellelements eine Untersetzung zwischenzuschalten.

Bei einer weiteren vorteilhaften Ausgestaltung der Erfindung ist die Kraft, die der Aktuator auf das Stellelement übertragt, von der Winkelposition des Stellelements bezogen auf eine Ruheposition oder Nullstellung des Stellelements abhängig. Insbesondere ist die Kraft bei einer größeren Abweichung der Winkelposition des Stellelements von der Ruheposition oder Nullstellung größer als bei einer geringeren Abweichung. Vorteilhafterweise wird eine Tabelle ("look-up table") bereitgestellt, die unterschiedlichen Winkelpositionen des Stellelements bestimmte Kräfte zuweist, insbesondere durch die Vorgabe eines Antriebsstroms für den Aktuator, die der Aktuator auf das Stellelement übertragen soll. Auf diese Weise wird bevorzugt eine von der momentanen Auslenkung abhängige Motorunterstützung für den Anwender realisiert.

Bei einer weiteren vorteilhaften Ausgestaltung des eingangs genannten Endoskops ist mindestens ein Winkelmesser für eine berührungslose Erfassung ausgestaltet, insbesondere sind Winkelmesser für eine berührungslose Erfassung der Winkelpositionen von Bedienelement und Stellelement ausgestaltet. In dieser Ausbildung wird auch unabhängig von der Positionierung der ersten und zweiten Rotationsachse zueinander eine erfinderische Weiterbildung des Stands der Technik gesehen, das heißt, auch bei einer Positionierung der ersten und zweiten Rotationsachse im rechten Winkel zueinander. Alle oben beschriebenen vorteilhaften Ausgestaltungen lassen sich auch bei dieser berührungslosen Erfassung der Winkelpositionen realisieren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: ein Ausführungsbeispiel eines flexiblen Endoskops mit einem ablenkbaren distalen Abschnitt,
- Fig. 2: das Endoskop gemäß Fig. 1 in schematischer teilweiser Schnittdarstellung,
- Fig.3: eine detaillierte Darstellung von Elementen des Endoskops aus Fig. 1,
- Fig. 4: ein erstes Ausführungsbeispiel einer Koppeleinrichtung aus Fig. 3,
- Fig. 5: ein zweites Ausführungsbeispiel einer Koppeleinrichtung aus Fig. 3,
- Fig. 6: die Koppeleinrichtung aus Fig. 5 bei einer Verlagerung des ersten Teils,
- Fig. 7: die Koppeleinrichtung gemäß Fig. 6 nach der resultierenden Verlagerung des zweiten Teils,
- Fig. 8: eine weiteres Ausführungsbeispiel in der Draufsicht,
- Fig. 9: das Ausführungsbeispiel gemäß Fig. 8 in der Seitenansicht,
- Fig. 10: noch ein weiteres Ausführungsbeispiel in der Draufsicht, und
- Fig. 11: das Ausführungsbeispiel gemäß Fig. 10 in der Seitenansicht.

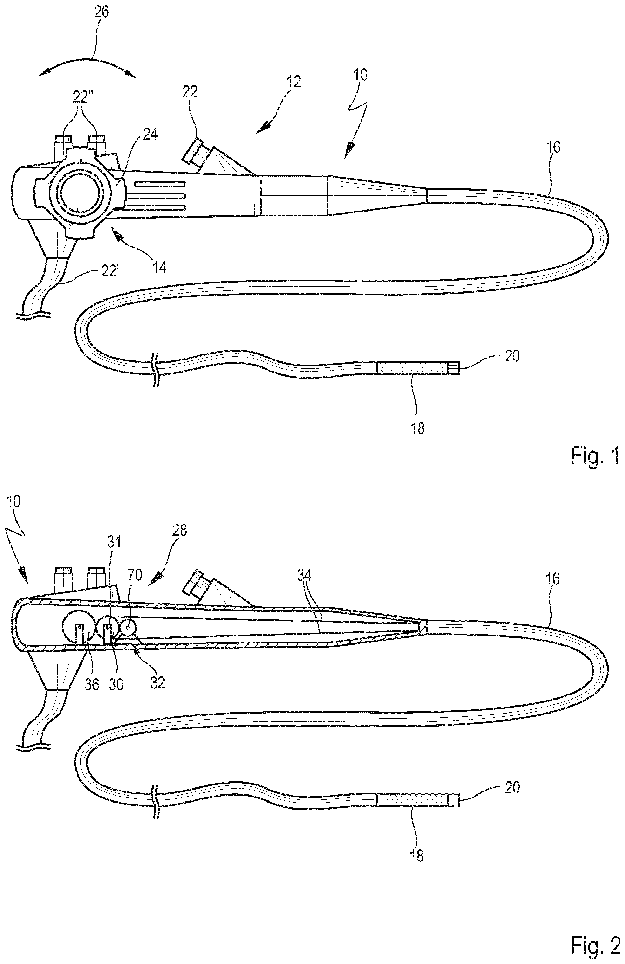

Fig. 1 zeigt ein endoskopisches Instrument 10 mit einem Bedienstück 12, mit einem Bedienelement 14 und einem flexiblen Endoskopschaft 16. Es sei darauf hingewiesen, dass die Erläuterungen im Rahmen der Erfindung in gleicher Weise für einen starren Instrumentenschaft (nicht gezeigt) zutreffen.

Das endoskopische Instrument 10 wird zu Untersuchungs- und/oder Operationszwecken in medizinischen Verfahren verwendet. In dem Instrumentenschaft 16 verlaufen eine nicht dargestellte Endoskopoptik in Form von Lichtleitfasern, Bildleitern, verschiedene Kanäle, wie ein Saug- und ein Spülkanal, und ein Instrumentenkanal. Der Instrumentenschaft 16 ist proximal mit dem Bedienstück 12 verbunden und erstreckt sich distal bis in einen ablenkbaren Abschnitt 18, bei dem es sich hier insbesondere um einen Endabschnitt handelt.

Dieser weist ein Endstück 20 in Form einer Abschlussbuchse auf. Das Endstück 20 ist der Bereich des Endoskopschafts 16, in dem die Lichtleitfasern, die Bildleiter und Kanäle enden. Der Endoskopschaft 16 ist in den Figuren nur teilweise dargestellt. Das Bedienstück 12 weist einen Anschluss 22, ein Versorgungskabel 22' und Tasten 22" auf. Der Anschluss 22 führt zu einem Instrumentenkanal. Durch ihn können Instrumente in den Endoskopschaft 16 und bis durch das Endstück 20 hindurch geführt werden. Dadurch können im Bereich vor dem Endstück 20 beispielsweise Operationen durchgeführt werden.

Das Versorgungskabel 22' beinhaltet verschiedene Arten von Versorgungen, wie beispielsweise eine elektrische Versorgung, Lichtleiter, Saug- und Spülschläuche und/oder Datenleitungen. Die Bildgebung des endoskopischen Instruments 10 erfolgt über einen nicht dargestellten Bildsensor im Inneren des Bedienstücks 12 oder im Endstück 20, wobei die Bilddaten über das Versorgungskabel 22' nach außen geleitet werden, insbesondere zu einer nicht dargestellten Kamerasteuerungseinheit (camera control unit, CCU).

Für ein Steuern des Ablenkens des Abschnitts 18 ist das Bedienelement 14 vorgesehen, wobei hier deren Handhabe 24 zu sehen ist, der in die Richtungen des Doppelpfeils 26 gedreht werden kann. Durch Drehen des Bedienelements 14 wird der ablenkbare Abschnitt 18 auf/ab oder links/rechtsabgelenkt. Die jeweilige Ablenkung korrespondiert mit der Drehrichtung des Bedienelements 14, entweder gegen oder im Uhrzeigersinn.

Wie in Fig. 2 dargestellt ist, sind in einem proximalen Endbereich 28 des Bedienstücks 12 ein Stellelement 30 mit einer ersten Rotationsachse 31 sowie ein Aktuator 32 mit einer zweiten Rotationsachse 70 angeordnet. Das Stellelement 30 ist mit einem Zugelement 34 verbunden, das durch den Endoskopschaft 16 hindurchgeführt ist und sich bis in den Endabschnitt 18 hinein erstreckt. Das Zugelement 34 ist um das Stellelement 30 herum geführt und hier einstückig ausgebildet. Ferner ist im Bedienstück 12 ein Mitnehmerelement 36 gezeigt, das ein Teil des Bedienelements 14 ist.

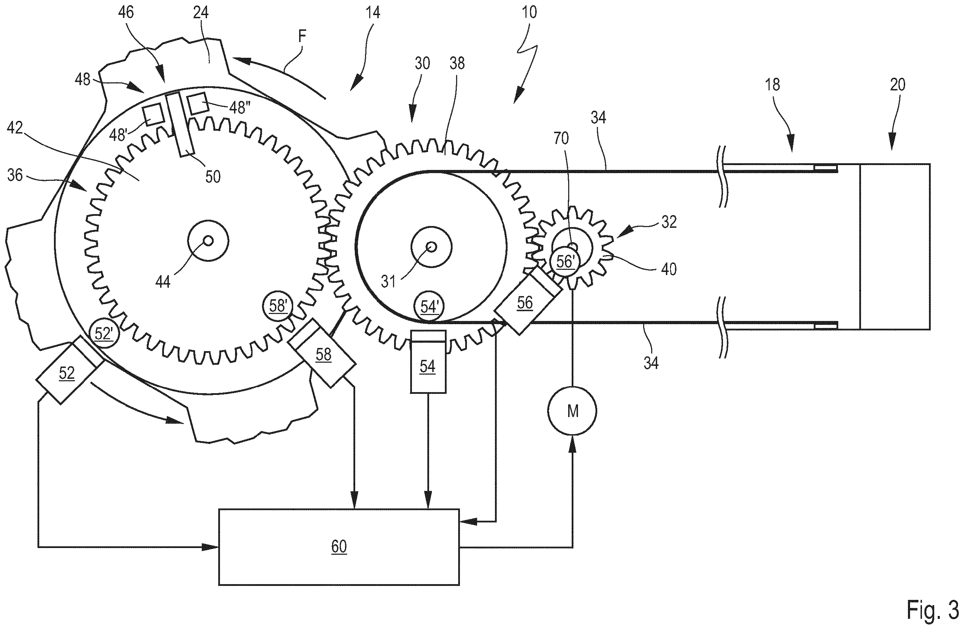

Fig. 3 zeigt eine detaillierte Ansicht von Elementen aus dem endoskopischen Instrument 10 aus Fig. 1. Es ist zu erkennen, dass das Stellelement 30 einen ersten Zahnkranz 38 aufweist, der Aktuator 32 einen zweiten Zahnkranz 40 aufweist, und der erste und der zweite Zahnkranz 38, 40 derart angeordnet sind, dass sie in Eingriff miteinander stehen. Ferner weist der Aktuator 32 einen Elektromotor M auf und weist das Bedienelement 14 einen dritten Zahnkranz 42 auf, wobei der erste und der dritte Zahnkranz 38, 42 derart angeordnet sind, dass sie in Eingriff miteinander stehen. Damit sind das Bedienelement 14 und der Aktuator 32 derart miteinander mechanisch wirkverbunden, dass die durch den Benutzer auf das Bedienelement 14 ausgeübte Kraft F zumindest teilweise auf den Aktuator 32 übertragen werden kann.

Die Handhabe 24 und das Mitnehmerelement 36 des Bedienelements 14 sind so angeordnet, dass sie relativ zueinander verlagert werden können. Die Handhabe 24 und das Mitnehmerelement 36 sind so miteinander gekoppelt, dass eine Verlagerung der Handhabe 24 eine Verlagerung des Mitnehmerelements 36 bewirken kann. Bei dieser Ausführungsform handelt es sich bei den Verlagerungen um rotatorische Verlagerungen.

Um dies zu erzielen, sind die Handhabe 24 und das Mitnehmerelement 36 entlang einer Welle 44 angeordnet, wobei sich die Handhabe 24 rotatorisch relativ zum Mitnehmerelement 36 und zur Welle 44 verlagern kann. Um die Verlagerung der Handhabe 24 und die Verlagerung des Mitnehmerelements 36 zu koppeln, weist das Bedienelement 14 eine Koppeleinrichtung 46 auf, die einen ersten Teil 48 und einen zweiten Teil 50 aufweist.

Bei der hier gezeigten Ausführungsform ist der erste Teil 48 fest mit der Handhabe 24 verbunden und ist der zweite Teil 50 fest mit dem Mitnehmerelement 36 verbunden. Die Teile 48, 50 sind so ausgebildet und angeordnet, dass eine Kraftübertragung zwischen Handhabe 24 und Mitnehmerelement 36 erfolgen kann. Der erste Teil 48 weist zwei Elemente 48', 48" auf, und der zweite Teil 50 ist als Vorsprung ausgebildet, der zwischen den zwei Elementen 48', 48" angeordnet ist. Dabei sind der erste und der zweite Teil 48, 50 zueinander so angeordnet, dass eine Verlagerung des ersten Teils 48 eine Verlagerung des zweiten Teils 50 bewirken kann.

Ein erster Winkelmesser 52 ist dafür ausgebildet, eine Indikation über einen ersten Stellwinkel des Bedienelements 14 bzw. der Handhabe 24 zu geben. Ein zweiter Winkelmesser 54 ist dafür ausgebildet, eine Indikation über einen zweiten Stellwinkel des Stellelements 30 zu geben. Ein dritter Winkelmesser 56 ist dafür ausgebildet, eine Indikation über einen dritten Stellwinkel des Aktuators 32 zu geben. Ein vierter Winkelmesser 58 ist dafür ausgebildet, eine Indikation über einen vierten Stellwinkel des Mitnehmerelements 36 zu geben.

Bei der hier gezeigten Ausführungsform sind die Winkelmesser 52, 54, 56, 58 als Hall-Sensoren ausgeführt, wobei lediglich symbolisch ein jeweils zugeordneter Magnet 52', 54', 56', 58' dargestellt ist. Die Hall-Sensoren sind ein Beispiel für eine berührungslose Winkelmessung, die auch in Alleinstellung eine Weiterentwicklung gegenüber dem Stand der Technik darstellt. Die Winkelerfassung kann auch auf andere Weise berührungslos erfolgen, insbesondere kapazitiv oder induktiv.

Das endoskopische Instrument 10 weist ferner eine Steuereinrichtung 60 auf, die wahlweise für eine, mehrere oder alle der folgenden Funktionen ausgebildet ist:
- Der Aktuator 32 wird unter Berücksichtigung des ersten Stellwinkels und des zweiten Stellwinkels angesteuert.
- Der Aktuator 32 wird unter Berücksichtigung des dritten Stellwinkels und des vierten Stellwinkels angesteuert.
- Der Aktuator 32 wird unter Berücksichtigung des ersten Stellwinkels und des vierten Stellwinkels angesteuert.
- Der Aktuator 32 wird so angesteuert, dass das Stellelement 30 in einer vorgegebenen Position gehalten wird, wenn der Benutzer keine Kraft F auf das Bedienelement 14 ausübt.
- Der Aktuator 32 wird so angesteuert, dass er auf das Stellelement 30 eine Kraft ausübt, die zumindest bereichsweise zumindest ungefähr proportional zu der Kraft F ist, die der Benutzer auf das Bedienelement 14 ausübt.

Die grundlegende Funktionsweise ist wie folgt. Es sei angenommen, dass der Benutzer eine Kraft F auf die Handhabe 24 entgegen dem Uhrzeigersinn (aus der Betrachtungsperspektive) aufbringt. Die Steuereinrichtung 60 detektiert eine Veränderung des ersten Stellwinkels und/oder eine Differenz zwischen dem ersten und dem zweiten Stellwinkel. So wird erkannt, dass der Benutzer eine Kraft F auf die Handhabe 24 ausübt. Zumindest ein Teil der Kraft F wird auf das Stellelement 30 übertragen.

Die Steuereinrichtung 60 erkennt, in welcher Richtung der Benutzer das Stellelement 30 verlagern möchte, hier mit dem Uhrzeigersinn, und steuert den Aktuator 32 über den Motor M entsprechend an. Hier bedeutet dies, dass der dritte Zahnkranz 42 entgegen dem Uhrzeigersinn angetrieben wird.

Im Falle eines Ausfalls des Aktuators 32 überträgt der erste Teil 48 weiterhin zumindest einen Teil der Kraft F auf den zweiten Teil 50, so dass der Teil der Kraft F auf das Stellelement 30 übertragen wird.

Es sei an dieser Steller erneut darauf hingewiesen, dass bei bevorzugten Ausgestaltungen das Bedienelement eine erste Handhabe aufweist, die ein erstes Stellelement verlagert, und eine zweite Handhabe, die ein zweites Stellelement verlagert. Dabei führen eine Betätigung der ersten Handhabe zu einer ersten Bewegung des Abschnitts, insbesondere zu einer Bewegung links/rechts, und eine Betätigung der zweiten Handhabe zu einer zweiten Bewegung des Abschnitts, insbesondere zu einer Bewegung auf/ab. Bevorzugt ist die eine Handhabe auf einer Hohlwelle angeordnet, in der eine Welle der anderen Handhabe angeordnet ist. Demnach können bevorzugte Ausgestaltungen auch zwei oder mehr Handhaben, Stellelemente und Aktuatoren aufweisen.

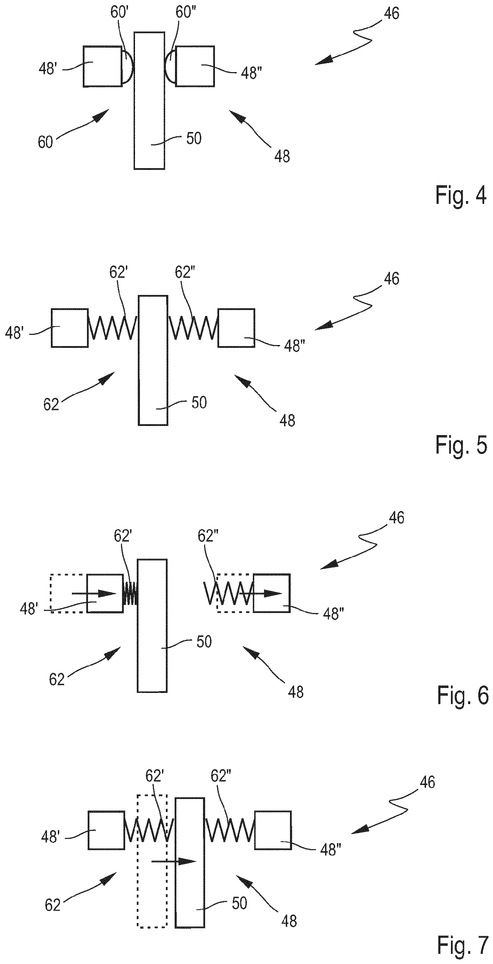

Fig. 4 zeigt eine zweite Ausführungsform einer Koppeleinrichtung 46 mit einer Kraftmesseinrichtung 60', 60". In einer bevorzugten Ausgestaltung zeigt die Kraftmesseinrichtung 60', 60" an, ob zwischen dem ersten Teil 48 und dem zweiten Teil 50 eine Kraft ausgeübt wird. In einer weiteren bevorzugten Ausgestaltung gibt die Kraftmesseinrichtung 60', 60" eine Indikation über die Größe einer zwischen dem ersten Teil 48 und dem zweiten Teil 50 ausgeübten Kraft. Bevorzugt stehen der erste Teil 48 und der zweite Teil 50 über die Kraftmesseinrichtung 60', 60" unmittelbar in Kontakt, so dass der Benutzer bei einer Betätigung des Bedienelements 14 eine Rückmeldung erhält.

Fig. 5 zeigt eine dritte Ausführungsform einer Koppeleinrichtung 46. Bei dieser Ausführungsform ist am ersten Teil eine Federeinrichtung 62 mit Federelementen 62', 62" angeordnet, so dass eine Kraft zwischen dem ersten Teil 48 und dem zweiten Teil 50 über die Federeinrichtung 62 übertragen wird.

Fig. 6 zeigt die Situation, bei der sich der erste Teil 48 gegenüber dem zweiten Teil 50 verschiebt. Das zweite Federelement 62" wird dabei gespannt, so dass der Benutzer eine Rückmeldung erhält. Der Aktuator 32 wird wiederum aufgrund der vorher genannten Winkelinformation gesteuert.

Fig. 7 zeigt wieder die Ruheposition, nachdem das Mitnehmerelement 36 die Position eingenommen hat, die der Benutzer über die Handhabe 24 vorgegeben hat.

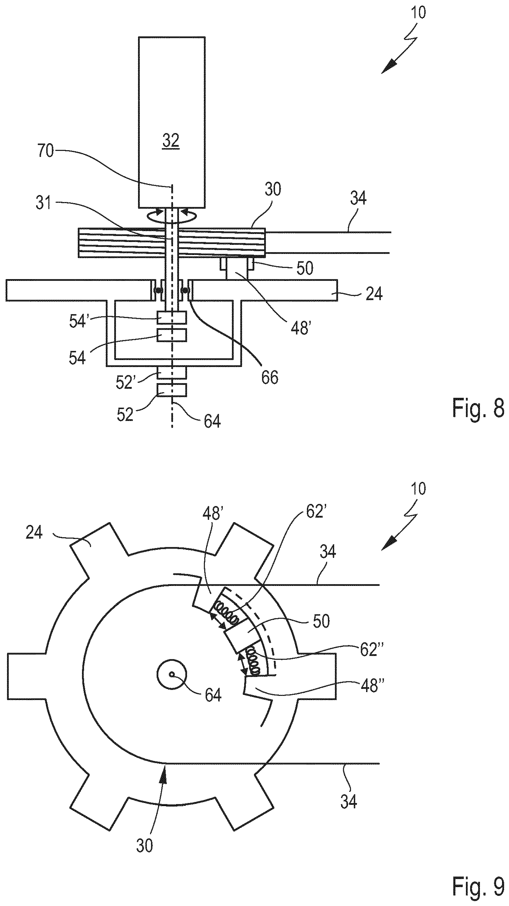

Fig. 8 zeigt ein weiteres Ausführungsbeispiel, bei dem die erste und zweite Rotationsachse zusammenfallen, indem der Aktuator 32 mit dem Stellelement 30 starr auf einer gemeinsamen Achse 64 gekoppelt ist. Die bisherigen Bezugszeichen und die Erläuterungen zu diesen Elementen gelten entsprechend auch hier. Im Unterschied zum Ausführungsbeispiel gemäß Fig. 3 werden hier keine Zahnkränze benötigt. Außerdem ist die Handhabe 24 mittels eines Lagers 66 ebenfalls auf der Achse 64 angeordnet.

Fig. 9 zeigt das Ausführungsbeispiel gemäß Fig. 8 in der Seitenansicht.

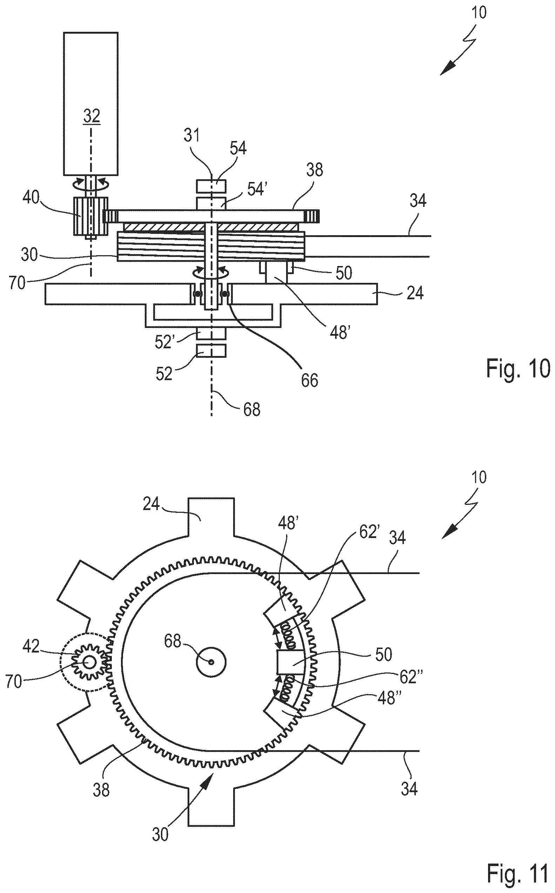

Fig. 10 zeigt noch ein weiteres Ausführungsbeispiel, bei dem eine Handhabe 24 und das Stellelement 30 auf einer gemeinsamen Achse 68 angeordnet sind, die von einer Rotationsachse 70 des Aktuators 32 verschieden ist. Diese Ausgestaltung ist einfach zu realisieren, bietet aber dennoch die Möglichkeit einer Untersetzung zwischen dem Aktuator 32 und dem Stellelement 30, hier zwischen dem ersten Zahnkranz 38 und dem zweiten Zahnkranz 40. Die erste und zweite Rotationsachse 31, 70 verlaufen hier zumindest im Wesentlichen parallel.

Fig. 11 zeigt das Ausführungsbeispiel gemäß Fig. 10 in der Seitenansicht.

## Patentansprüche

1. Endoskopisches Instrument (10) mit
einem Bedienstück (12) mit einem Bedienelement (14),
einem Instrumentenschaft (16) mit einem betätigbaren Abschnitt (18), wobei der Instrumentenschaft (16) mit dem Bedienstück (12) verbunden ist,
einem Stellelement (30) mit einer ersten Rotationsachse (31),
einem Zugelement (34), das mit dem Stellelement (30) und dem Abschnitt (18) des Instrumentenschafts (16) mechanisch gekoppelt ist, so dass eine rotatorische Verlagerung des Stellelements (30) eine Betätigung des Abschnitts (18) mittels einer Kraftübertragung über das Zugelement (34) bewirken kann, und
einem Aktuator (32), der mit dem Stellelement (30) gekoppelt ist, so dass eine Betätigung des Aktuators (32) die Verlagerung des Stellelements (30) durch Kraftübertragung vom Aktuator (32) auf das Stellelement (30) bewirken kann, wobei der Aktuator (32) eine zweite Rotationsachse (70) aufweist,
wobei das Bedienelement (14) dafür ausgebildet ist, dass ein Benutzer durch Betätigen des Bedienelements (14) die Verlagerung des Stellelements (30) bewirken kann,
wobei das Bedienelement (14) mit dem Stellelement (30) derart mechanisch gekoppelt ist, dass eine durch den Benutzer auf das Bedienelement (14) ausgeübte Kraft (F) zumindest teilweise auf das Stellelement (30) übertragen werden und die Verlagerung des Stellelements (30) bewirken kann, und
wobei die erste Rotationsachse (31) und die zweite Rotationsachse (70) in einem Winkel kleiner 90° zueinander stehen,
**dadurch gekennzeichnet, dass** das Bedienelement (14) eine Handhabe (24) und ein Mitnehmerelement (36) aufweist, das Mitnehmerelement (36) auf einer Welle angeordnet ist, die Handhabe (24) entlang der Welle des Mitnehmerelements (36) oder konzentrisch zu einer gedachten Verlängerung dieser Welle angeordnet ist, die Handhabe (24) und das Mitnehmerelement (36) so angeordnet sind, dass sie rotatorisch relativ zueinander verlagert werden können, die Handhabe (24) und Mitnehmerelement (36) so miteinander gekoppelt sind, dass eine rotatorische Verlagerung der Handhabe (24) eine rotatorische Verlagerung des Mitnehmerelements (36) bewirken kann, und das Mitnehmerelement (36) mit dem Stellelement (30) gekoppelt ist, und wobei das Bedienelement (14) eine Koppeleinrichtung (46) aufweist, die einen ersten Teil (48) und einen zweiten Teil (50) aufweist, wobei einer der Teile (48, 50) mit einer Handhabe (24) verbunden ist und der andere Teil (50, 48) mit dem Mitnehmerelement (36) verbunden ist, wobei die Teile (48, 50) so ausgebildet und angeordnet sind, dass bei der rotatorischen Verlagerung der Handhabe (24) eine Kraftübertragung zwischen der Handhabe (24) und dem Mitnehmerelement (36) erfolgen kann, wobei der erste Teil (48) zwei Elemente (48', 48") aufweist und der zweite Teil (50) als Vorsprung ausgebildet ist, der zwischen den zwei Elementen (48', 48") angeordnet ist, und der erste und der zweite Teil (48, 50) zueinander so angeordnet sind, dass eine rotatorische Verlagerung des ersten Teils (48) eine rotatorische Verlagerung des zweiten Teils (50) bewirken kann.

2. Endoskopisches Instrument (10) mit
einem Bedienstück (12) mit einem Bedienelement (14),
einem Instrumentenschaft (16) mit einem betätigbaren Abschnitt (18), wobei der Instrumentenschaft (16) mit dem Bedienstück (12) verbunden ist,
einem Stellelement (30) mit einer ersten Rotationsachse (31),
einem Zugelement (34), das mit dem Stellelement (30) und dem Abschnitt (18) des Instrumentenschafts (16) mechanisch gekoppelt ist, so dass eine rotatorische Verlagerung des Stellelements (30) eine Betätigung des Abschnitts (18) mittels einer Kraftübertragung über das Zugelement (34) bewirken kann, und
einem Aktuator (32), der mit dem Stellelement (30) gekoppelt ist, so dass eine Betätigung des Aktuators (32) die Verlagerung des Stellelements (30) durch Kraftübertragung vom Aktuator (32) auf das Stellelement (30) bewirken kann, wobei der Aktuator (32) eine zweite Rotationsachse (70) aufweist,
wobei das Bedienelement (14) dafür ausgebildet ist, dass ein Benutzer durch Betätigen des Bedienelements (14) die Verlagerung des Stellelements (30) bewirken kann,
wobei das Bedienelement (14) mit dem Stellelement (30) derart mechanisch gekoppelt ist, dass eine durch den Benutzer auf das Bedienelement (14) ausgeübte Kraft (F) zumindest teilweise auf das Stellelement (30) übertragen werden und die Verlagerung des Stellelements (30) bewirken kann, und
wobei die erste Rotationsachse (31) und die zweite Rotationsachse (70) in einem Winkel kleiner 90° zueinander stehen,
**dadurch gekennzeichnet, dass** das Bedienelement (14) eine Koppeleinrichtung (46) aufweist, die einen ersten Teil (48) und einen zweiten Teil (50) aufweist, wobei einer der Teile (48, 50) mit einer Handhabe (24) verbunden ist und der andere Teil (50, 48) mit dem Stellelement (30) verbunden ist, wobei die Teile (48, 50) so ausgebildet und angeordnet sind, dass die Handhabe (24) und das Stellelement (30) rotatorisch relativ zueinander verlagert werden können und bei einer rotatorischen Verlagerung der Handhabe (24) eine Kraftübertragung zwischen der Handhabe (24) und dem Stellelement (30) erfolgen kann, wobei der erste Teil (48) zwei Elemente (48', 48") aufweist und der zweite Teil (50) als Vorsprung ausgebildet ist, der zwischen den zwei Elementen (48', 48") angeordnet ist, und der erste und der zweite Teil (48, 50) zueinander so angeordnet sind, dass eine rotatorische Verlagerung des ersten Teils (48) eine rotatorische Verlagerung des zweiten Teils (50) bewirken kann.

3. Endoskopisches Instrument nach einem der vorhergehenden Ansprüche, wobei das Stellelement (30) einen ersten Zahnkranz (38) aufweist, der Aktuator (32) einen zweiten Zahnkranz (40) aufweist und der erste und der zweite Zahnkranz (38, 40) kraftschlüssig gekoppelt sind, insbesondere derart angeordnet sind, dass sie im Eingriff miteinander stehen.

4. Endoskopisches Instrument nach einem der vorhergehenden Ansprüche, wobei das Stellelement (30) einen ersten Zahnkranz (38) aufweist, das Bedienelement (14) einen dritten Zahnkranz (42) aufweist, und der erste und der dritte Zahnkranz (38, 42) kraftschlüssig gekoppelt sind, insbesondere derart angeordnet sind, dass sie im Eingriff miteinander stehen.

5. Endoskopisches Instrument nach einem der vorhergehenden Ansprüche, wobei das Bedienelement (14) und der Aktuator (32) derart miteinander mechanisch wirkverbunden sind, dass die durch den Benutzer auf das Bedienelement (14) ausgeübte Kraft (F) zumindest teilweise auf den Aktuator (32) übertragen werden kann.

6. Endoskopisches Instrument nach einem der vorhergehenden Ansprüche, wobei die Koppeleinrichtung (46) eine Kraftmesseinrichtung (60) aufweist, die dafür ausgebildet ist, anzuzeigen, ob eine Kraft zwischen dem ersten Teil (48) und dem zweiten Teil (50) ausgeübt wird, oder eine Indikation über die Größe einer zwischen dem ersten Teil (48) und dem zweiten Teil (50) ausgeübten Kraft zu geben, insbesondere auch eine Indikation über die Richtung der ausgeübten Kraft zu geben.

7. Endoskopisches Instrument nach einem der vorhergehenden Ansprüche, wobei am ersten und/oder am zweiten Teil (48, 50) eine Federeinrichtung (62) angeordnet ist, so dass eine Kraft zwischen dem ersten Teil und dem zweiten Teil (48, 50) über die Federeinrichtung (62) übertragen wird.

8. Endoskopisches Instrument nach einem der vorhergehenden Ansprüche, ferner mit einem ersten Winkelmesser (52), der dafür ausgebildet ist, eine Indikation über einen ersten Stellwinkel einer Handhabe (24) des Bedienelements (14) zu geben und/oder mit einem zweiten Winkelmesser (54), der dafür ausgebildet ist, eine Indikation über einen zweiten Stellwinkel eines Stellelements (30) zu geben.

9. Endoskopisches Instrument nach einem der vorhergehenden Ansprüche, ferner mit einer Steuereinrichtung (60), die dafür ausgebildet ist, unter Berücksichtigung eines ersten Stellwinkels einer Handhabe (24) des Bedienelements (14) und eines zweiten Stellwinkels des Stellelements (30), den Aktuator (32) anzusteuern.

10. Endoskopisches Instrument nach einem der vorhergehenden Ansprüche, ferner mit einer Steuereinrichtung (60), die dafür ausgebildet ist, den Aktuator (32) so anzusteuern, dass das Stellelement (30) in einer vorgegebenen Position gehalten wird, wenn der Benutzer keine Kraft (F) auf das Bedienelement (14) ausübt.

11. Endoskopisches Instrument nach einem der vorhergehenden Ansprüche, ferner mit einer Steuereinrichtung (60), die dafür ausgebildet ist, den Aktuator (32) so anzusteuern, dass er auf das Stellelement (30) eine Kraft ausübt, die zumindest bereichsweise zumindest ungefähr proportional zu der Kraft (F) ist, die der Benutzer auf das Bedienelement (14) ausübt.

12. Endoskopisches Instrument nach einem der vorhergehenden Ansprüche, wobei die erste und zweite Rotationsachse (31, 70) zusammenfallen, insbesondere der Aktuator (32) mit dem Stellelement (30) starr auf einer gemeinsamen Achse (64) gekoppelt ist.

13. Endoskopisches Instrument nach einem der Ansprüche 1 bis 11, wobei eine Handhabe (24) und das Stellelement (30) auf einer gemeinsamen Achse (68) angeordnet sind, die insbesondere von einer Rotationsachse (70) des Aktuators (32) verschieden ist.

## Claims

1. Endoscopic instrument (10) comprising
a manipulating piece (12) having a manipulating element (14),
an instrument shaft (16) having a section (18) to be manipulated by a user, wherein the instrument shaft 16) is coupled to the manipulating piece (12),
a positioning element (30) having a first rotational axis (31),
a pulling element (34) which is mechanically coupled to the positioning element (30) and the section (18) of the instrument shaft (16), such that a rotational displacement of the positioning element (30) may achieve an actuation of the section (18) by transmitting a force via the pulling element (34),
an actuator (32) coupled to the positioning element (30), such that an actuation of the actuator (32) may provide for a displacement of the positioning element (30) by transmitting a force from the actuator (32) to the positioning element (30), wherein the actuator (32) comprises a second rotational axis (70),
wherein the manipulating element (14) is configured such that a user may displace the positioning element (30) by actuating the manipulating element (14),
wherein the manipulating element (14) is mechanically coupled to the positioning element (30) such that a force (F) exerted by the user onto the manipulating element (14) may at least be partially transferred onto the positioning element (30) and may provide for a displacement of the positioning element (30), and
wherein die first rotational axis (31) and the second rotational axis (70) are arranged at an angle of less than 90° relative to one another,
**characterized in that** the manipulating element (14) comprises a handle (24) and an engaging element (36), the engaging element (36) being arranged on a shaft, the handle (24) being arranged along the shaft of the engaging element (36) or concentrically along a virtual extension of this shaft, the handle (24) and the engaging element (36) being arranged such that they can be rotationally displaced relatively to one another, and the handle and the engaging element are coupled with one another such that a rotational displacement of the handle (24) may result in a rotational displacement of the engaging element (36), and the engaging element (36) being coupled to the positioning element (30), and wherein the manipulating element (14) comprises a coupling arrangement (46) having a first part (48) and a second part (50), wherein one of the parts (48, 50) is connected to the handle (24) and the other part (50, 48) is connected to the engaging element (36), wherein the parts (48, 50) are configured and arranged such that a transmission of a force between the handle (24) and the engaging element (36) may be effected when the handle (24) is rotationally displaced, wherein the first part (48) comprises two elements (48', 48") and the second part (50) is embodied as a projection being arranged between the two elements (48', 48"), and the first and the second parts (48, 50) are arranged relative to one another such that a rotational displacement of the first part (48) may result in a rotational displacement of the second part (50).

2. Endoscopic instrument (10) comprising
a manipulating piece (12) having a manipulating element (14),
an instrument shaft (16) having a section (18) to be manipulated by a user, wherein the instrument shaft 16) is coupled to the manipulating piece (12),
a positioning element (30) having a first rotational axis (31),
a pulling element (34) which is mechanically coupled to the positioning element (30) and the section (18) of the instrument shaft (16), such that a rotational displacement of the positioning element (30) may achieve an actuation of the section (18) by transmitting a force via the pulling element (34),
an actuator (32) coupled to the positioning element (30), such that an actuation of the actuator (32) may provide for a displacement of the positioning element (30) by transmitting a force from the actuator (32) to the positioning element (30), wherein the actuator (32) comprises a second rotational axis (70),
wherein the manipulating element (14) is configured such that a user may displace the positioning element (30) by actuating the manipulating element (14),
wherein the manipulating element (14) is mechanically coupled to the positioning element (30) such that a force (F) exerted by the user onto the manipulating element (14) may at least be partially transferred onto the positioning element (30) and may provide for a displacement of the positioning element (30), and
wherein die first rotational axis (31) and the second rotational axis (70) are arranged at an angle of less than 90° relative to one another,
**characterized in that** the manipulating element (14) comprises a coupling arrangement (46) having a first part (48) and a second part (50), wherein one of the parts (48, 50) is connected to a handle (24) and the other part (50, 48) is connected to the positioning element (30), wherein the parts (48, 50) are configured and arranged such that the handle (24) and the positioning element (30) may be rotationally displaced relative to each other and when rotationally displacing the handle (24) a transmission of a force between the handle (24) and the positioning element (30) may be effected, wherein the first part (48) comprises two elements (48', 48") and the second part (50) is embodied as a projection being arranged between the two elements (48', 48"), and the first and the second parts (48, 50) are arranged relative to one another such that a rotational displacement of the first part (48) may result in a rotational displacement of the second part (50).

3. Endoscopic instrument according to any preceding claim, wherein the positioning element (30) comprises a first gear ring (38), the actuator (32) comprises a second gear ring (40), and the first and second gear rings (38, 40) are coupled by force-fit, in particular are arranged such that the teeth are meshing.

4. Endoscopic instrument according to any preceding claim, wherein the positioning element (30) comprises a first gear ring (38), the manipulating element (14) comprises a third gear ring (42), and first and third gear rings (38, 42) are coupled by force-fit, in particular are arranged such that their teeth are meshing.

5. Endoscopic instrument according to any preceding claim, wherein the manipulating element (14) and the actuator (32) are mechanically operationally coupled to one another such the force (F) exerted by the user onto the manipulating element (14) may at least be partially transmitted onto the actuator (32).

6. Endoscopic instrument according to any preceding claim, wherein the coupling arrangement (46) comprises a force measuring device (60) configured to indicate whether a force is being exerted between the first part (48) and the second part (50) or to provide an indication related to the magnitude of a force exerted between the first part (48) and the second part (50), in particular including an indication regarding the direction of the force being exerted.

7. Endoscopic instrument according to any preceding claim, wherein a spring device (62) is arranged at the first and/or the second part (48, 50) such that a force between the first and the second part (48, 50) is transmitted via the spring device (62).

8. Endoscopic instrument according to any preceding claim, further comprising a first angle measuring device (52) embodied to provide an indication of a first angle position of a handle (24) of the manipulating element (14) and/or a second angle measuring device (54) embodied to provide an indication of a second angle position of a positioning element (30).

9. Endoscopic instrument according to any preceding claim, further comprising a control device (60) configured to drive the actuator (32) taking into account a first angle position of a handle (24) of the manipulating element (14) and a second angle position of a positioning element (30).

10. Endoscopic instrument according to any preceding claim, further comprising a control device (60) configured to drive the actuator (32) such that the positioning element (30) is maintained at a given position if the user does not exert a force (F) on the manipulating element (14).

11. Endoscopic instrument according to any preceding claim, further comprising a control device (60) configured to drive the actuator (32) such that it exerts a force onto the positioning element (30) which is at least in certain ranges at least approximately proportional to the force (F) which the user exerts onto the manipulating element (14).

12. Endoscopic instrument according to any preceding claim, wherein the first and second rotational axes (31, 70) coincide, and in particular the actuator (32) is fixedly coupled with the positioning element (30) on a common axis (64).

13. Endoscopic instrument according to any of claims 1 to 11, wherein a handle (24) and the positioning element (30) are arranged on a common axis (68) which, in particular, is different from the rotational axis (70) of the actuator (32).

## Revendications

1. Instrument endoscopique (10) avec
- une pièce de commande (12) avec un élément de commande (14),
- une tige d'instrument (16) avec une partie actionnable (18), dans lequel la tige d'instrument (16) est reliée à la pièce de commande (12),
- un élément de réglage (30) avec un premier axe de rotation (31),
- un élément de traction (34), qui est couplé mécaniquement à l'élément de réglage (30) et à la partie (18) de la tige d'instrument (16), de telle manière qu'un déplacement rotatif de l'élément de réglage (30) puisse provoquer un actionnement de la partie (18) au moyen d'une transmission de force par l'intermédiaire de l'élément de traction (34), et
- un actionneur (32), qui est couplé à l'élément de réglage (30), de telle manière qu'un actionnement de l'actionneur (32) puisse provoquer le déplacement de l'élément de réglage (30) par transmission de force de l'actionneur (32) à l'élément de réglage (30), dans lequel l'actionneur (32) présente un second axe de rotation (70),
- dans lequel l'élément de commande (14) est configuré de telle façon qu'un utilisateur puisse provoquer le déplacement de l'élément de réglage (30) par actionnement de l'élément de commande (14),
- dans lequel l'élément de commande (14) est couplé mécaniquement à l'élément de réglage (30), de telle manière qu'une force (F) exercée par l'utilisateur sur l'élément de commande (14) puisse être transmise au moins partiellement à l'élément de réglage (30) et provoquer le déplacement de l'élément de réglage (30), et
- dans lequel le premier axe de rotation (31) et le second axe de rotation (70) forment entre eux un angle inférieur à 90°,
**caractérisé en ce que**
l'élément de commande (14) présente une poignée (24) et un élément d'entraînement (36), l'élément d'entraînement (36) est disposé sur un arbre, la poignée (24) est disposée le long de l'arbre de l'élément d'entraînement (36) ou concentriquement à un prolongement virtuel de cet arbre,
la poignée (24) et l'élément d'entraînement (36), sont disposés de telle manière qu'ils puissent être déplacés en rotation l'un par rapport à l'autre,
la poignée (24) et l'élément d'entraînement (36) sont couplés l'un à l'autre de telle manière qu'un déplacement rotatif de la poignée (24) puisse provoquer un déplacement rotatif de l'élément d'entraînement (36), et l'élément d'entraînement (36) est couplé à l'élément de réglage (30), et
dans lequel l'élément de commande (14) présente un dispositif de couplage (46), qui présente une première partie (48) et une seconde partie (50),
dans lequel une des parties (48, 50) est reliée à une poignée (24) et l'autre partie (50, 48) est reliée à l'élément d'entraînement (36),
dans lequel les parties (48, 50) sont configurées et disposées de telle manière que, lors du déplacement rotatif de la poignée (24), il puisse se produire une transmission de force entre la poignée (24) et l'élément d'entraînement (36),
dans lequel la première partie (48) présente deux éléments (48', 48") et la seconde partie (50) est réalisée sous forme de saillie, qui est disposée entre les deux éléments (48', 48"), et la première et la seconde parties (48, 50) sont disposées l'une par rapport à l'autre de telle manière qu'un déplacement rotatif de la première partie (48) puisse provoquer un déplacement rotatif de la seconde partie (50).

2. Instrument endoscopique (10) avec
- une pièce de commande (12) avec un élément de commande (14),
- une tige d'instrument (16) avec une partie actionnable (18), dans lequel la tige d'instrument (16) est reliée à la pièce de commande (12),
- un élément de réglage (30) avec un premier axe de rotation (31),
- un élément de traction (34), qui est couplé mécaniquement à l'élément de réglage (30) et à la partie (18) de la tige d'instrument (16), de telle manière qu'un déplacement rotatif de l'élément de réglage (30) puisse provoquer un actionnement de la partie (18) au moyen d'une transmission de force par l'intermédiaire de l'élément de traction (34), et
- un actionneur (32), qui est couplé à l'élément de réglage (30), de telle manière qu'un actionnement de l'actionneur (32) puisse provoquer le déplacement de l'élément de réglage (30) par transmission de force de l'actionneur (32) à l'élément de réglage (30), dans lequel l'actionneur (32) présente un second axe de rotation (70),
- dans lequel l'élément de commande (14) est configuré de telle façon qu'un utilisateur puisse provoquer le déplacement de l'élément de réglage (30) par actionnement de l'élément de commande (14),
- dans lequel l'élément de commande (14) est couplé mécaniquement à l'élément de réglage (30), de telle manière qu'une force (F) exercée par l'utilisateur sur l'élément de commande (14) puisse être transmise au moins partiellement à l'élément de réglage (30) et provoquer le déplacement de l'élément de réglage (30), et
- dans lequel le premier axe de rotation (31) et le second axe de rotation (70) forment entre eux un angle inférieur à 90°,
**caractérisé en ce que**
l'élément de commande (14) présente un dispositif de couplage (46), qui présente une première partie (48) et une seconde partie (50),
dans lequel une des parties (48, 50) est reliée à une poignée (24) et l'autre partie (50, 48) est reliée à l'élément de réglage (30),
dans lequel les parties (48, 50) sont configurées et disposées de telle manière que la poignée (24) et l'élément de réglage (30) puissent être déplacés en rotation l'un par rapport à l'autre et que lors d'un déplacement rotatif de la poignée (24), il puisse se produire une transmission de force entre la poignée (24) et l'élément de réglage (30),
dans lequel la première partie (48) présente deux éléments (48', 48") et la seconde partie (50) est réalisée sous forme de saillie, qui est disposée entre les deux éléments (48', 48"), et la première et la seconde parties (48, 50) sont disposées l'une par rapport à l'autre de telle manière qu'un déplacement rotatif de la première partie (48) puisse provoquer un déplacement rotatif de la seconde partie (50).

3. Instrument endoscopique selon l'une quelconque des revendications précédentes, dans lequel l'élément de réglage (30) présente une première couronne dentée (38), l'actionneur (32) présente une deuxième couronne dentée (40) et la première et la deuxième couronnes dentées (38, 40) sont couplées par force, en particulier sont disposées de telle manière qu'elles engrènent l'une avec l'autre.

4. Instrument endoscopique selon l'une quelconque des revendications précédentes, dans lequel l'élément de réglage (30) présente une première couronne dentée (38), l'élément de commande (14) présente une troisième couronne dentée (42), et la première et la troisième couronnes dentées (38, 42) sont couplées par force, en particulier sont disposées de telle manière qu'elles engrènent l'une avec l'autre.

5. Instrument endoscopique selon l'une quelconque des revendications précédentes, dans lequel l'élément de commande (14) et l'actionneur (32) sont mécaniquement en liaison active l'un avec l'autre, de telle manière que la force (F) exercée sur l'élément de commande (14) par l'utilisateur puisse être au moins partiellement transmise à l'actionneur (32).

6. Instrument endoscopique selon l'une quelconque des revendications précédentes, dans lequel le dispositif de couplage (46) présente un dispositif de mesure de force (60), qui est configuré pour afficher si une force est exercée entre la première partie (48) et la seconde partie (50), ou pour donner une indication sur la grandeur d'une force exercée entre la première partie (48) et la seconde partie (50), en particulier aussi pour donner une indication sur la direction de la force exercée.

7. Instrument endoscopique selon l'une quelconque des revendications précédentes, dans lequel un dispositif de ressort (62) est disposé sur la première et/ou sur la seconde partie (48, 50), de telle manière qu'une force soit transmise entre la première partie et la seconde partie (48, 50) au moyen du dispositif de ressort (62).

8. Instrument endoscopique selon l'une quelconque des revendications précédentes, en outre avec un premier goniomètre (52), qui est configuré pour donner une indication sur un premier angle de réglage d'une poignée (24) de l'élément de commande (14) et/ou avec un second goniomètre (54), qui est configuré pour donner une indication sur un second angle de réglage d'un élément de réglage (30).

9. Instrument endoscopique selon l'une quelconque des revendications précédentes, en outre avec un dispositif de commande (60), qui est configuré pour commander l'actionneur (32) en tenant compte d'un premier angle de réglage d'une poignée (24) de l'élément de commande (14) et d'un second angle de réglage de l'élément de réglage (30).

10. Instrument endoscopique selon l'une quelconque des revendications précédentes, en outre avec un dispositif de commande (60), qui est configuré pour commander l'actionneur (32) de telle manière que l'élément de réglage (30) soit maintenu dans une position prédéterminée, lorsque l'utilisateur n'exerce aucune force (F) sur l'élément de commande (14).

11. Instrument endoscopique selon l'une quelconque des revendications précédentes, en outre avec un dispositif de commande (60), qui est configuré pour commander l'actionneur (32) de telle manière qu'il exerce sur l'élément de réglage (30) une force qui est au moins localement au moins environ proportionnelle à la force (F), que l'utilisateur exerce sur l'élément de commande (14).

12. Instrument endoscopique selon l'une quelconque des revendications précédentes, dans lequel le premier et le second axes de rotation (31, 70) coïncident, en particulier l'actionneur (32) est couplé avec l'élément de réglage (30) de façon rigide sur un axe commun (64).

13. Instrument endoscopique selon l'une quelconque des revendications 1 à 11, dans lequel une poignée (24) et l'élément de réglage (30) sont disposés sur un axe commun (68), qui est différent en particulier d'un axe de rotation (70) de l'actionneur (32).
